# EUROPEAN PATENT SPECIFICATION

(11) **EP 1 180 243 B1**
(45) Date of publication and mention of the grant of the patent: **20.07.2005**
(21) Application number: 00935321.0
(22) Date of filing: 23.05.2000
(51) Int. Cl.: G01N 33/574, A61K 48/00, A61K 38/17, A61P 35/00, A61K 38/53

(54) **USES OF NOTCH RELATED GENES**
VERWENDUNG VON NOTCH VERWANDTEN GENEN
UTILISATION DE G NES DU TYPE NOTCH

(30) Priority: 26.05.1999 GB 9912132
(43) Date of publication of application: 20.02.2002
(73) Proprietor: The University of Manchester, Manchester M13 9PL (GB)
(72) Inventor: BARON, Martin, Manchester M25 3JA (GB)
(74) Representative: Banford, Paul Clifford
(86) International application number: PCT/GB2000/001990
(87) International publication number: WO 2000/073329

(56) References cited:
- WO-A-97/18822
- WO-A-97/37223
- WO-A-98/57621
- FOSTIER MAGGY ET AL: "Genetic characterization of the Drosophila melanogaster suppressor of deltex gene: A regulator of notch signaling." GENETICS, vol. 150, no. 4, December 1998 (1998-12), pages 1477-1485, XP002152975 ISSN: 0016-6731
- GRAY GRACE E ET AL: "Human ligands of the Notch receptor." AMERICAN JOURNAL OF PATHOLOGY, vol. 154, no. 3, March 1999 (1999-03), pages 785-794, XP000960906 ISSN: 0002-9440
- PIROZZI GREGORIO ET AL: "Identification of novel human WW domain-containing proteins by cloning of ligand targets." JOURNAL OF BIOLOGICAL CHEMISTRY, vol. 272, no. 23, 1997, pages 14611-14616, XP002152977 ISSN: 0021-9258

## Description

The present invention relates to the use of human homologues of a *Drosophila* Notch regulator gene and the encoded protein products thereof, as well as derivatives, fragments and analogues thereof and antibodies thereto in diagnosis and therapy.

During development, the regulation and integration of cell to cell signalling pathways co-ordinates the programme of events that specify the differentiation of different cell types. Notch is a transmembrane receptor protein which mediates a signal that controls the timing and outcome of cell differentiation decisions. Inappropriate activation of Notch has been associated with oncogenesis in humans and mice, causing T-Cell lymphoma and mammary gland tumours. In addition mutations in human Notch genes may result in the genetically inherited disease CADASIL, (Cerebral Autosomal Dominant Arteriopathy with Sub-cortical Infarcts and Leukoencephalopathy) which leads to a predisposition to multiple strokes and early onset dementia.

The analysis of genetic interactions has proven a valuable tool for isolating components or regulators of signalling pathways. By identifying and mapping mutations that interact with Notch mutant phenotypes components or regulators of the Notch pathway may be identified. One candidate for a regulator of the Notch pathway in *Drosophila* is Suppressor of deltex (Su(dx)). As its name suggests, Su(dx) mutations suppress the phenotypes resulting from mutations in deltex. Deltex is a protein that enhances the activity of the Notch pathway. The *Drosophila* mutations Su(dx)¹ and Su(dx)² were originally described as second chromosome mutants that dominantly suppressed the deltex phenotype. More recently another allele has been identified, Su(dx)^{sp}.

Over expression of *Drosophila* Su(dx) results in over proliferation and cell differentiation phenotypes. It is likely that more than one target protein is recognised by Su(dx) and that multiple pathways are regulated by its activity leading to the regulation of cell differentiation and cell proliferation. Genetic data in *Drosophila* indicates that one of these regulated pathways is the Notch signalling pathway where Su(dx) acts as a negative regulator of the signal. In particular, genetic data indicates that Su(dx) regulates Notch signalling during the differentiation of *Drosophila* wing veins. The cell proliferation phenotype resulting from over expression of Su(dx) may reflect regulation of additional signals as well as or instead of Notch.

The inventor has cloned the *Drosophila* Suppressor of deltex (Su(dx)) gene and the gene has been sequenced and the sequence of its protein product Su(dx) determined. Details of the cloning are provided in Cornell *et al*., Genetics 152: 567-576 (June 1999). Such details were fully described in GB patent application no. 9912132.9, which serves as priority for this application.

The inventor used his *Drosophila* Su(dx) clone to screen a DNA sequence database and the clone with the sequence with the closest homology with the *Drosophila* Su(dx) clone obtained (SEQ ID NO. 1) and used to screen a human foetal liver cDNA library using standard methods. The clone (SEQ ID NOS. 2 and 3) obtained by this screen was used to screen a human breast tumour cDNA library using standard methods and a clone (SEQ ID NO. 4) obtained that corresponds to the whole open reading frame expected for a sequence homologous to the Drosophila Su(dx) gene. The nucleic acid molecule having sequence homology to Drosophila Su(dx) obtained was termed clone h-SDRP-1 as is shown as SEQ ID No. 4. The protein sequence encoded by clone h-SDRP-1 is shown as SEQ ID No.5.

The h-SDRP protein was analysed and found to have the same domain architecture as Drosophila Su(dx) in that it contains a C-terminal HECT domain (or E3 ubiquitin ligase domain) and four WW domains.

A protein with substantial homology with h-SDRP is described in International application no. PCT/US97/05547 which described several polypeptides having WW domains, whose sequence was determined by screening cDNA libraries with a synthetic peptide based upon a consensus motif for WW proteins. PCT/US97/05547 does not suggest any function for the polypeptides described therein.

Gray *et al*., discloses three human homologues of the Notch ligands Delta and Serrate, i.e. the genes *HJ1, HJ2,* and *H-Delta-1*. (Gray *et al*. (1999), Human Ligands of the Notch Receptor, *American Journal of Pathology*, **154** (3):785-793). However, none of the three genes disclosed in this publication have any relationship with the human suppressor of deltex related protein. The DNA and protein sequences are totally dissimilar from each other

Work carried out by Fostier *et al*. discloses that the *Drosophila* suppressor of deltex gene *Su(dx)* is a negative modifier of the Notch signal transduction pathway.(Fostier *et al*. ((1998), Genetic Characterization of the *Drosophila melanogaster* Suppressor of *deltex* Gene: A regulator of Notch Signaling, *Genetics,* **150**:1477-1485).

However, none of the prior art discloses a method of *in vitro* screening by means of assaying for the expression of of a nucleic acid encoding a human suppressor of deltex related protein or for the concentration of the protein itself. Furthermore, neither Gray *et al*. (1999) nor Fostier *et al*. (1998) disclose a medical use of the modulator of human suppressor of deltex related protein.

The present invention in its first aspect provides for the use of h-SDRP nucleic acids, proteins and antibodies in diagnosis.

The h-SDRP nucleic acid and amino acid sequences and antibodies thereto may be used according to the invention for the detection and quantitation of h-SDRP mRNA and protein, to study the expression thereof, to produce h-SDRP proteins, fragments and other derivatives or analogues for use in the study, assay and manipulation of differentiation and other physiological processes in which *Drosophila* Su(dx) is postulated to be involved, e.g. diseases of cell fate and abnormal differentiation and cell proliferation.

It is preferred that the h-SDRP nucleic acid has at least 60% sequence homology with the h-SDRP sequence provided as SEQ ID NO. 4(nucleic acid) or SEQ ID NO. 5 (protein), more preferably at least 70%, even more preferably at least 80% and most preferably at least 90% sequence homology with the h-SDRP nucleic acid sequence provided as SEQ ID NO. 4 or h-SDRP protein sequence provided as SEQ ID NO. 5 or subfragments or variants thereof specific to the h-SDRP sequence.

The h-SDRP used according to the present invention preferably retain *Drosophila* Su(dx)-function, i.e. they are capable of displaying one or more known functions of the *Drosophila* Su(dx) protein, for example the protein can suppress deltex function, as shown by providing a transgenic *Drosophila* which expresses the vertebrate Su(dx) like protein and observing whether the expression can replace mutations that knock out endogenous *Drosophila* Su(dx). The phenotype of the *Drosophila* could be studied to see if over expression of the vertebrate Su(dx)-like nucleic acid causes epithelial cell over-proliferation or wing vein differentiation phenotypes similar to those found on over expression of *Drosophila* Su(dx).

The h-SDRP nucleic acid molecules, proteins and antibodies may, but not necessarily disrupt Notch function by contributing to abnormal Notch signalling. Consequently, the h-SDRP nucleic acid molecules, proteins and antibodies may be used in methods of diagnosis and treatment of diseases of cell fate, differentiation and proliferation which may involve inappropriate Notch signalling.

Due to the degeneracy of the genetic code it is clear that the sequence of h-SDRP could be changed substantially without effecting the sequence of the protein encoded thereby, to provide a variant of h-SDRP. Suitable nucleotide variants of SEQ ID NO. 4 are those having a sequence altered by the substitution of different codons that encode the same or a functionally equivalent amino acid residue within the sequence, thus producing a silent change. Other suitable variants are those having nucleotide sequences homologous to SEQ ID NO. 4 but comprising all or portions of sequence which arc altered by the substitution of different codons that encode an amino acid of similar polarity to the amino acid it substitutes, to produce a silent change. Codons for an amino acid of a specific type or function may be substitutes to encode a different amino acid within the same class. For example non polar (hydrophobic amino acids include alanine, leucine, isoleucine, valine, proline, phenylalanine, tryptophan and methionine. The polar neutral amino acids include glycine, serine, threonine, cysteine, tyrosine, asparagine and glutamine. The positively charged (basic) amino acids include lysine, arginine and histidine. The negatively charged (acidic) amino acids include aspartic acid and glutamic acid.

Suitable h-SDRP protein variants of SEQ ID NO. 5 are those having sequences in which substitutions of amino acids have been made with amino acids of the same function, thus producing a silent change. Amino acids of a specific type or function may be substitutes to encode a different amino acid within the same class. For example non polar (hydrophobic amino acids include alanine, leucine, isoleucine, valine, proline, phenylalanine, tryptophan and methionine. The polar neutral amino acids include glycine, serine, threonine, cysteine, tyrosine, asparagine and glutamine. The positively charged (basic) amino acids include lysine, arginine and histidine. The negatively charged (acidic) amino acids include aspartic acid and glutamic acid.

Further modifications in the protein sequence of SEQ ID NO. 5 are also envisaged, i.e. those which occur during or after translation, e.g. by acetylation, amidation, carboxylation, phosphorylation, proteolytic cleavage or linkage to a ligand.

It has been suggested in the literature that certain diseases of cell fate and abnormal differentiation are due to inappropriate Notch signalling. Such diseases include oncogenesis, dementia and CADASIL. Furthermore, many cancerous and hyperproliferative conditions are said to exhibit upregulated Notch, such as colon cancer, cervical cancer, breast cancer, squamous adenocarcinoma, seminoma, melanoma, and lung cancer, as well as other hyperproliferative disorders and these may be diagnosed by the expression of the Su(dx)-like gene. Other disorders that involve the proliferation and differentiation state of cells that may also involve Notch regulation include wound healing, rheumatoid arthritis and vascular diseases such as arteriosclerosis.

International application no. PCT/US98/27610 describes several polynucleotides which are differentially expressed between cancerous and non-cancerous tissues and therefore which may be involved in abnormal cell differentiation. Although three of the sequences disclosed do contain one WW domain they are not homologous with h-SDRP.

The inventor has found that h-SDRP nucleic acid molecules are expressed more in breast tumour tissue than in tissues from healthy patients, resulting in more of the h-SDRP protein being present. Consequently the inventor proposes that the expression of h-SDRP may be used as a marker for breast cancer.

The inventor has also found that h-SDRP nucleic acid molecules are expressed in a cell culture model of angiogenesis which suggests that h-SDRP is involved in the angiogenesis process. Consequently the inventor proposes that the over expression of h-SDRP may be used as a marker for increased angiogenesis. Such a marker may indicate the presence of tumour tissue as increased angiogenesis is involved in providing capillaries to tumours.

According to a second aspect of the invention there is provided a method of screening for breast cancer comprising assaying for the expression of h-SDRP nucleic acid.

According to a third aspect of the present invention there is provided a method of screening for increased angiogenesis comprising assaying for the over expression of h-SDRP nucleic acid.

As both breast cancer and tumour have previously been proposed to be diseases potentially related or caused by abnormal Notch signalling, the inventor proposes that his finding could be extrapolated to other diseased said to be related or caused by abnormal Notch signalling.

Accordingly, the fourth aspect of the invention provides a method of screening for diseases associated with abnormal Notch signalling comprising assaying for the expression of h-SDRP nucleic acid.

Such diseases include oncogenesis, dementia and CADASIL. Furthermore, many cancerous and hyperproliferative conditions are said to exhibit upregulated Notch, such as colon cancer, cervical cancer, breast cancer, squamous adenocarcinoma, seminoma, melanoma, and lung cancer, as well as other hyperproliferative disorders and these may be diagnosed by the expression of Su(dx). Other disorders that involve the proliferation and differentiation state of cells that may also involve Notch regulation include wound healing, rheumatoid arthritis and vascular diseases such as arteriosclerosis.

It may be that persons with an inherited disease including breast cancer and diseases associated with abnormal Notch signalling has over expression of h-SDRP nucleic acid molecule before the disease develops. Therefore the presence of h-SDRP nucleic acid may be an indication of a genetically inherited predisposition to a disease of cell fate, proliferation or differentiation, such as breast cancer.

Assaying for the expression of the h-SDRP according to the invention may be carried out in various ways that would be apparent to a person skilled in the art.

The identification of genetic or biochemical markers in blood or tissues that will detect cancer at early stages is a major goal of cancer research as it would allow early diagnosis and monitoring of the effectiveness of any treatment.

Currently, many methods of cancer diagnosis require tissue samples and are therefore invasive. Although h-SDRP may be used to screen for cancers in biopsy tissues it is preferred that said screening is carried out in a non-invasive manner.

By using h-SDRP as a tumour marker, particularly for breast cancer, allows classification of the tumour to allow different diagnosis and treatment of different degrees and types of cancer. Two classifications widely used in oncology that can benefit from identification of the expression levels of h-SDRP are staging of the cancerous disorder and grading the nature of the cancerous tissue. Details of both staging and grading in relation to cancer will be apparent to those skilled in the art.

The h-SDRP nucleic acid molecules, proteins or antibodies (or fragments or variants thereof) may be used according to the invention to screen the blood or tissues of patients to determine if that patient has breast cancer. It is further proposed that the nucleic acid molecules, proteins or antibodies (or fragments or variants thereof) may be used to screen the blood or tissues of patients to determine if that patient has a disease or disorder associated with abnormal differentiation or cellular proliferation due to inappropriate Notch signalling.

For screening, h-SDRP nucleic acid molecules, including homologous nucleic acid sequences and subsequences, including complementary sequences, can be used in Northern assays, dot blots and *in situ* hybridisation assays.

For example h-SDRP nucleic acid sequences or subsequences thereof comprising about at least 8 nucleotides specific to h-SDRP can be used as hybridisation probes. Preferably, the probes comprise at least 12 nucleotides specific to h-SDRP. More preferably, the probes comprise at least 15 nucleotides specific to h-SDRP. Even more preferably, the probes comprise at least 18 nucleotides specific to h-SDRP. Most preferably, the probes comprise at least 22 nucleotides specific to h-SDRP. Hybridisation assays using these probes can be used to detect, prognose, diagnose or monitor breast tumours.

Furthermore it is proposed that hybridisation assays using the above probes can be used to detect, prognose, diagnose or monitor conditions, disorders or disease states associated with changes in Notch activity or the change in expression of h-SDRP.

In particular, a hybridisation assay may be carried out by a method comprising contacting a sample containing nucleic acid with a h-SDRP nucleic acid probe capable of hybridising to substantially only h-SDRP DNA or mRNA under conditions such that hybridisation can occur and detecting or measuring any resultant hybridisation.

Another example of an assay for the expression of h-SDRP nucleic acid is a PCR assay using PCR primers specific for h-SDRP to amplify expression of this nucleic acid molecule. The amplified DNA may then be assayed by size analysis, for example by electrophoresis.

h-SDRP nucleic acid molecules particularly useful in the invention are the h-SDRP clone sequences provided as SEQ ID No. 1, SEQ ID No. 2 and SEQ ID No. 4. Any of these sequences or fragments thereof specific to h-SDRP may be used as hybridisation probes in the hybridisation method described above. Also the sequences may be used as targets against which specific PCR primers may be designed for use in the PCR assay described above.

Examples of suitable PCR primers specific for h-SDRP nucleic acid are:

PCR forward primer 5' GAA AGA GGG AAT CGT GTC TTA C 3' (SEQ ID No. 6)

PCR reverse primer 5' CGA TCA TCA ACT CTT CTT TCC C 3' (SEQ ID No. 7)

In a preferred assay for screening for expression of a h-SDRP nucleic acid molecule in tissue samples, a clone 8IIIA having the whole sequence provided as SEQ ID No. 2 was used to generate radiolabelled RNA for *in situ* hybridisation with tissue samples.

In another preferred assay for screening for expression of a h-SDRP nucleic acid molecule in cell culture samples, a clone S112506 having the whole sequence provided as SEQ ID No. 1 was used a to screen the Southern blot of cDNA prepared from the 3' end of RNA expressed during angiogenesis.

The h-SDRP proteins, variants, derivatives and fragments thereof, and antibodies thereto can be used in assays, such as immunoassays, to detect, prognose, diagnose or monitor various conditions, diseases and disorders associated with abnormal Notch function or to monitor the treatment thereof.

In particular, an immunoassay may be carried out by a method comprising contacting a sample derived from a patient with an anti-h-SDRP antibody under conditions such that immunospecific binding can occur and detecting or measuring the amount of immunospecific binding by the antibody.

The immunoassays which can be used include but are not limited to competitive and non-competitive assay systems using techniques such as western-blots (followed by sodium dodecyl sulphate polyacrylamide gel electrophoresis), immunocytochemistry,, radioimmunoassays, ELISA (enzyme linked immunosorbant assay), "sandwich" immunoassays, immunoprecipitation assays and fluorescent immunoassays

The assays according to the invention may be performed on patient tissue samples (e.g., from biopsy tissue), bodily fluids or on cell cultures derived from patient cells. Preferably the assay may be performed on breast tissue biopsy sample to diagnose breast cancer.

Furthermore it is proposed that an agent that modulates expression or activity of h-SDRP nucleic acid may be used to treat breast cancer and inhibit or stimulate angiogenesis, and by extrapolation, conditions associated with abnormal Notch function.

The invention will now be described by was of example only with reference to the following figures in which:

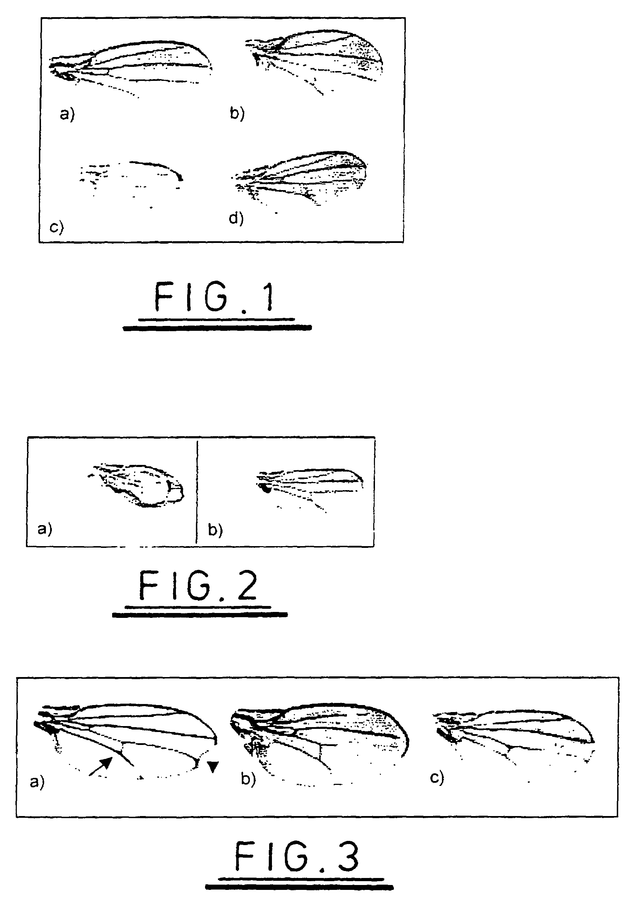

Figure 1 shows that compared to wild type (a), over-expression of Drosophila Su(dx) in the developing wing causes broadening of the wing through over-proliferation (b). Expression of a D-HECT form (c) and h-SDRP(d) give a small wing phenotype suggesting that both constructs act as dominant negatives for Su(dx) function when expressed in the Drosophila wing.
Figure 2 illustrates a) Co -expression of wild type Drosophila Su(dx) and D-HECT (a mutant form of Su(dx) lacking the C-terminal ubiquitin ligase domain), gave a small wing. This shows that the D-HECT form can act as dominant negative since the wing growth phenotype of wild type Su(dx) is blocked. (b) Similarly co-expression of Su(dx) and h-SDRP blocks the wing growth phenotype of Su(dx) indicating that the human protein also acts as a dominant negative for Su(dx). This is consistent with a conservation of specificity between Su(dx) and h-SDRP but indicates that when
Figure 3 shows that expression of h-SDRP alters Notch pathway signalling. (a) *nd,* a recessive *Notch* mutation displaying phenotypes characteristic of reduced Notch signalling I.e. wing margin loss (arrowhead) and vein thickening (arrow). (b) In the background of *Su(dx)* loss of function, *nd* displays a switch in phenotype resulting vein gaps that are characteristic of Notch activation phenotype. This phenotype only appears in the combination of both *nd* and *Su(dx)* mutations and thus indicates a functional relationship between these two genes.. (b) Expression of h-SDRP in the developing wing, in a *nd* mutant fly also produces vein gaps. These data further support the conclusion that, specificity of target selection is conserved between *Drosophila melanogaster* Su(dx) and h-SDRP and that expression of the human protein can regulate the Notch signalling pathway

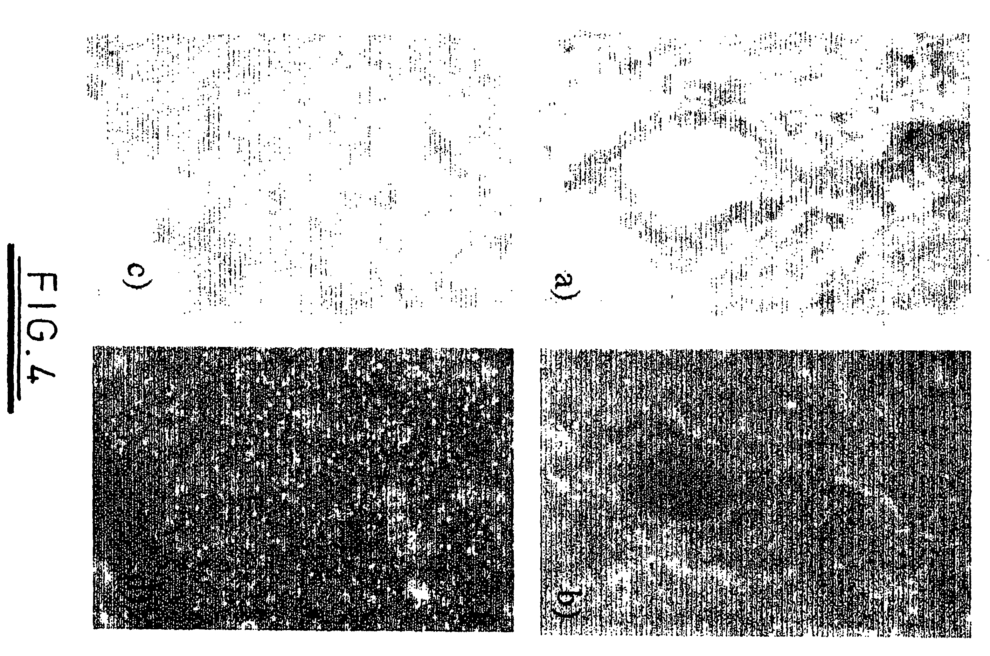

Figure 4 shows the results of hybridisation of a labelled h-SDRP RNA clone fragment of SEQ ID No 3. with breast tissue biopsy samples, showing in (a) and (c) the expression of h-SDRP in tumour tissue and in (b) and (d) the lack of expression of h-SDRP.

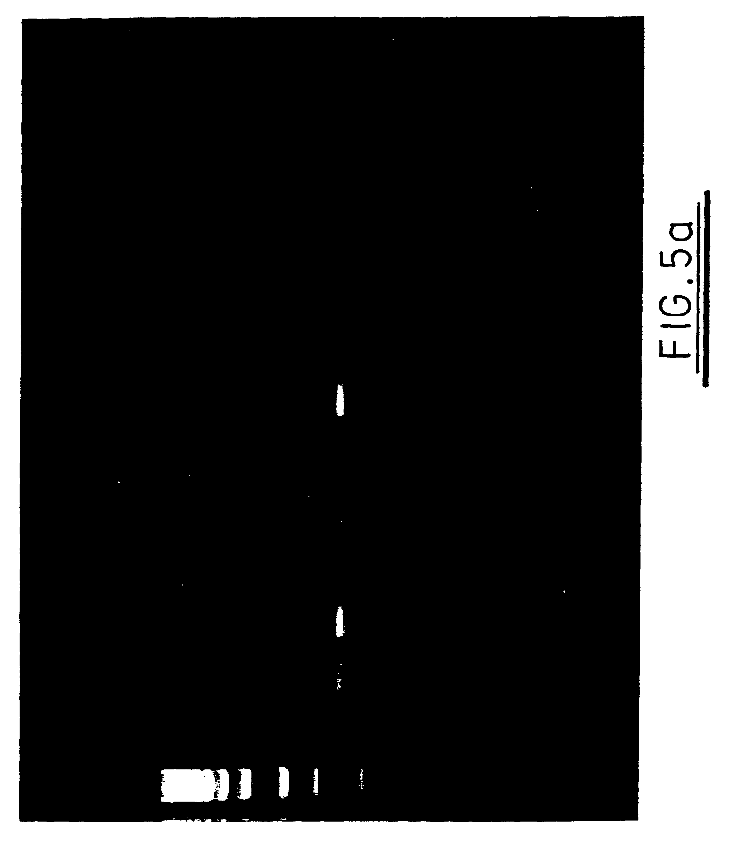

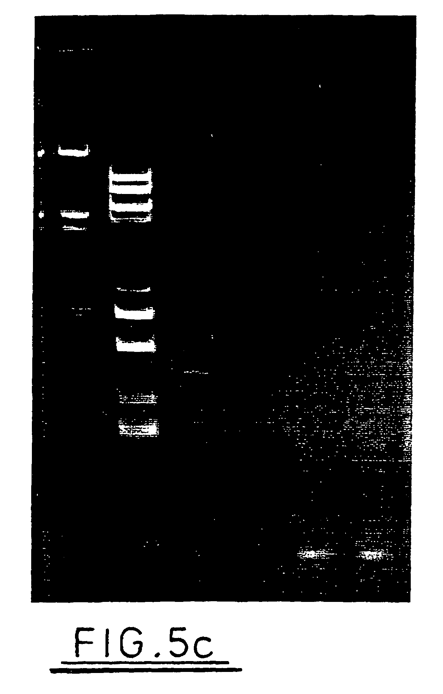

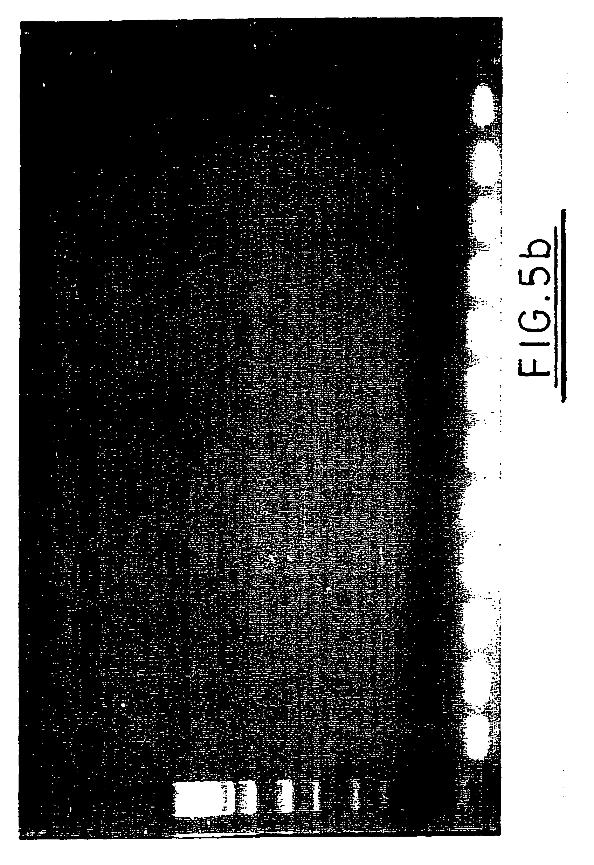

Figure 5 shows the results of reverse transcriptase PCR carried out on sample from breast tissue.

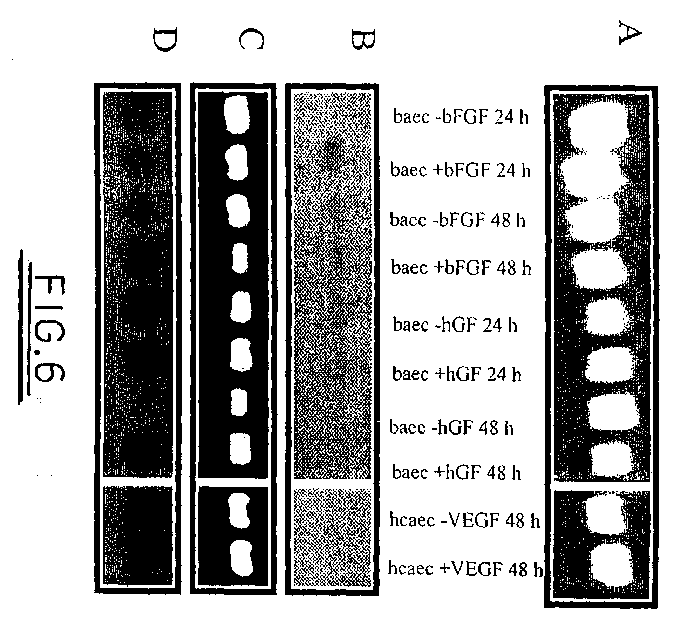

Figure 6 shows the results of probing endothelial cell RNA with a probe comprising the 3' end of the h-SDRP clone having SEQ ID No. 5.

### EXAMPLES

The inventor has cloned the *Drosophila melangaster* Suppressor of deltex (Su(dx)) gene and the gene has been sequenced and the sequence of its protein product Su(dx) determined. Details of the cloning are provided in Cornell *et al*., Genetics 152: 567-576 (June 1999). Such details were fully described in GB patent application no. 9912132.9, which serves as priority for this application.

The Drosophila Su(dx) clone was used to isolate a human Su(dx) clone. The human Su(dx) clone and fragments thereof was subsequently used to detect Su(dx) expression in both breast cancer biopsy tissue and endothelial cell cultures induced to have angiogenesis. Normal breast biopsy tissue and non-induced endothelial cells were shown not to express Su(dx) at detectable levels.

### 1. Cloning of the Drosophila Su(dx) gene

See Cornell *et al*., Genetics 152: 567-576 (June 1999) which is incorporated herein in its entirety by reference.

### 2. Cloning the human Su(dx)-like gene

The *Drosophila* Su(dx) gene clone sequence was used to search a DNA sequence database. The sequence with the closest homology with the *Drosophila* Su(dx) clone was a human expressed sequence tag clone S112506 (SEQ ID NO 1). The observed homology was to the HECT (or E3 ubiqutin liagase) domain region of Su(dx).

The S112506 clone was freely available from the Washington University EST project and was obtained.

A 1.3 kb insert from the S112506 clone was cut out, radiolabelled and used to screen a foetal liver cDNA library using standard methods. The library was a gift from G. Brady (School of Biological Sciences, University of Manchester).

A clone was detected and purified after 3 rounds of library screening. This clone was 8IIIa. This clone contains approximately 1.3 kb of sequence which overlaps that of S 112506. As expected, clone 8IIIa showed significant homology to Drosophila Su(dx) and is shown as SEQ ID No. 2. The protein sequence encoded by clone 8IIIa is shown as SEQ ID No.3.

The radiolabelled 1.3 kb insert from the 8IIIa clone was also used to screen a breast tumour cDNA library using standard methods. The clone obtained contains approximately 4.4 kb of sequence which corresponds to the whole open reading frame expected for a sequence homologous to the Drosophila Su(dx) gene. The nucleic acid molecule of human origin having sequence homology to Drosophila Su(dx) obtained was termed clone h-SDRP-1 and is shown as SEQ ID No. 4. The protein sequence encoded by clone h-SDRP-1 is shown as SEQ ID No.5.

### 2. Determining h-SDRP function

To investigate how h-SDRP function is related to *Drosophila* Su(dx), h-SDRP was expressed during *Drosophila* wing development and the phenotypes compared with those resulting from expression of wild type and a mutant form of *Drosophila* Su(dx).

### Construction of expression lines and methods:

Expression of wild type *Drosophila* Su(dx) in the developing wing has been described in Cornell *et al*., 1999 *supra*). To express h-SDRP in the *Drosophila* wing, the same protocol was followed. The cDNA of h-SDRP (SEQ ID NO. 4) was cloned into the multiple cloning site of vector pUAST (Brand and Perrimon 1993, Development 118:401-415). This places the hSDRP cDNA in front of a Gal4 transcription factor responsive promoter. A transgenic *Drosophila* line was then constructed using standard methodology (Brand and Perrimon 1993 *supra*) which carried the pUAST-hSDRP construct inserted into the *Drosophila* genome. h-SDRP expression was induced by crossing this line to the MS1096 *Drosophila* line (Cornell *et al*., 1999 *supra*) which expresses the Gal4 transcription factor in the developing wing.

Similarly a transgenic line was constructed to express a truncated *Drosophila* Su(dx) molecule which lacked the HECT ubiquitin ligase domain. This construct was predicted to act as a dominant negative against wild type Su(dx) because it retains the WW protein specificity domains but lacks the capability to ubquitinate and down regulate the target. Thus by binding to the target the D-HECT form is predicted to protect it from the activity of wild type Su(dx).

### Results of expression:

As reported in Cornell *et al*., 1999 *supra*, expression of *Drosophila* Su(dx) results in over-proliferation within the *Drosophila* wing leading to a broad wing phenotype (Figure 1b). When we expressed the D-HECT form of Drosophila Su(dx) we obtained a small wing phenotype, consistent with this construct operating as a dominant negative for Su(dx) activity (Figure lc). Similarly expression of h-SDRP also produced a small wing phenotype (Figure 1d) which was very similar to that observed from expression of the *Drosophila* D-HECT construct. This suggests that in the environment of the fly, the h-SDRP product acted as a dominant negative for Su(dx) activity. This would be expected if the targeting activity was intact but the enzymatic ubiquitin ligase function was working inefficiently. The latter could be due to the lower temperature of fly culture (25°C) compared to inside a human cell.

To demonstrate that both the D-HECT and h-SDRP acted as dominant negatives for wild type Su(dx) in *Drosophila*, we co-expressed in the same tissue both wild type Su(dx) and either D-HECT or h-SDRP. This was achieved by introducing into the same fly, the pUAST-Su(dx) and either pUAST D-HECT or pUAST-h-SDRP transgenes together with the MS1096-Gal4 expressing transgene, by crossing the individual lines together. The co-expression experiment showed that the expression of either D-HECT or the h-SDRP constructs blocked the wing growth phenotypes of wild type Su(dx) (Figure 2), thus confirming showing that both constructs acted as dominant negatives for Su(dx) activity. This implies that h-SDRP and Su(dx) are able to bind the same target molecules and supports the conclusion that the wild type function of h-SDRP is as a human homologue of Su(dx).

To provide further evidence that alteration of h-SDRP activity can modulate Notch receptor signalling, a genetic interaction experiment was performed. Expression of h-SDRP was induced in a fly carrying the recessive *notchoid* mutation of Notch. The *notchoid* mutation normally manifests phenotypes (loss of margin, thickened veins) which reflect a reduction of Notch signalling (Figure 3a and also see Fostier *et al*., 1998 Genetics 150:1477-1485). However when *Drosophila* Su(dx) activity is reduced by mutation, the *notchoid* phenotype is converted into one which reflects activation of the Notch pathway (wing vein gaps. Fig3b and see Fostier *et al*., 1998 *supra*). When h-SDRP was expresses in flies carrying the *notchoid* mutation we observed a similar switch to the vein gap phenotype, further supporting a Su(dx) related function for hSDRP (Fig3c).

### 3. Breast Tumour Biopsies

Radioactively labelled sense and anti-sense RNA was obtained by *in-vitro* transcription from T7 or T3 promoters of the cDNA. Radioactive labelling was by way of incorporation of ³²P labelled dUTP.

*In situ* hybridisation was carried out according to standard techniques. Tissue sections from breast biopsies were first pre-hybridised to block non-specific hybridisation. All sections were washed in DPEC water for 10 minutes then immersed in 0.2 M HCl for 20 mins, rinsed in two fold standard saline buffer (2x SSC) and then treated with I µg/ml proteinase K in 0.05M tris, pH 7.4 at 37°C for 60 mins. The slides were washed twice in solutions of glycine in PBS, again in PBS for 3 mins and then incubated in 4% paraformaldehyde in PBS for 20 mins. The slides were then immersed in a solution of freshly prepared 0.25% acetic anhydride in 0.1 M triethanolamine, pH 8.0 for 10 mins followed by rinses in 70% and 90% ethanol for 5 min. After air-drying for 1 hour 50µl of hybridisation solution was added to each section and left overnight at 37°C. The hybridisation solution containing 0.1 µg/ml of radiolabelled clone 8IIIa as a probe, 1 mg/ml BSA, 0.6 M NaCl, 50% deionised formamine, 10% w/v dextran sulphate, 0.2 mg/ml salmon sperm DNA, 0.02% w/v Ficoll, 0.02% w/v PVP, 10 mM tris-HCl (Ph 7.4) and 0.5 mM EDTA was heated to 100 oC for 5 mins and then rapidly cooled on ice.

After washing the sections twice in 0.5 x SSC with 10mM dithiothreitol for 5 mins and 0.5 x SSC with 1mM EDTA for 5 mins they were washed in a solution of 50% formamine, 0.15 NaCl, 5mM tris (pH 7.4), 0.5 mM EDTA for 10 mins. A further four washes in 0.5 x SSC at 55°C each of 5 mins was followed by washes in 0.5 x SSC at room temperature, again for 5 mins.

The slides were exposed at 4°C for 10 days in a light sealed box, developed with Kodak D-19 developer for 5 min and fixed for 5 min. Haematoxylin and eosin were used to counterstain the sections.

The fixed sections of tumour and non-tumour derived tissue were hybridised either with the sense or antisense 8IIIa RNA. The sense probe is a control for non-specific binding. The hybridisation of radioactive probes was detected after washing to remove unbound material, by overlaying the tissue with a photographic emulsion sensitive to the emitted radioactive particles. The presence and density of grains formed indicates the presence of the bound probe. The probe will bind where complementary RNA molecules are present. Thus the control sense strand probe should not bind.

The inventors have found that the antisense probe hybridised strongly to tumour-derived cells but not to normal breast tissue (see Fig 4). Probing with the control sense strand showed little or no signal with both normal and tumour derived breast tissue. This indicates a strong up-regulation of expression of h-SDRP in the breast tumours.

### 4. Expression of h-SDRP Protein in Breast Tissues

Reverse Transcriptase PCR (RT-PCR) was performed using a GIBCO-BRL *Superscript One Step* RT-PCR kit. Primers (SEQ ID Nos. 6 and 7) were specific for the 5' end of h-SDRP protein sequence.

0.5 microgram of RNA template (see below) was used in each reaction. cDNA synthesis was performed for 1 cycle at 50°C for 30 minutes followed by a denaturation step at 94°C for 2 minutes. PCR amplification was performed for 40 cycles consisting of 94°C denaturation (15 seconds), an annealing step of 55°C for 30 seconds and an elongation step of 72°C for 80 seconds. A final extension of 1 cycle of 72°C for 10 minutes was performed. RNA templates were obtained from Ann Canfield, Nigel Bundred and Richard Linforth (The Victoria University of Manchester).

RNA templates for RT-PCR were from N= normal tissue; T= tumour derived tissue; Nt= "normal tissue" taken from around outside of the tumour; D=DCIS, an early stage altered cell type not yet progressed to a full tumour; LN= metastatic tumour tissue taken from lymph node; B= metastaic tumour taken from bone; M= size markers.

The results of the RT-PCR are shown in Figure 5 a, b and c. Sets of samples which were derived from a particular individual are underlined. Presence of a band in Figure 5a indicates that h-SDRP mRNA was present in the sample. In Figure 5a bands can be seen in all of the tumour derived tissue, including the early stage pre-tumour (D). None of the normal breast tissue RNA (N) show any expression (samples derived from different individuals).

One of the samples derived from tissue which was surrounding the removed tissue (Nt patient 4) shows the presence of h-SDRP mRNA whereas the other Nt samples show no detectable expression. To investigate this further RT-PCR was carried out as above, but with primers to Glyceraldehyde-phosphate dehydrogenase (GAP). This is a commonly used positive control as the gene is widely expressed. The presence of a band in Figure 5b indicates that there is no problem with the quality of the RNA in the samples and that the absence of detectable h-SDRP mRNA in cannot be due to poor quality RNA in the N samples.

Figure 5c shows RT-PCR detection of h-SDRP mRNA in RNA from a breast tumour cell line (T47D) (lane 3) but not from RNA derived from normal breast tissue (lane 4). Lanes 5 and 6 are GAP controls for the respective RNAs. Lanes 1 and 2 are DNA size markers (M).

### 5. Angiogenesis and h-SDRP

A bovine endothelial in vitro angiogenesis model has been used to investigate whether h-SDRP plays a role in the angiogenic process. The experimental details are provided in full below. In brief, RNA was isolated from bovine endothelial cells and various stages of the angiogenic process brought on by the addition of different growth factors and the mRNA 3' end sequences converted to cDNA and blotted on to membranes following the method of Brady *et al*., Current Biology 5: 909-922 (1995). The cDNA on the membranes were then blotted using a 3' end specific probe to the h-SDRP gene. The strength of the signal obtained reflects the proportion of h-SDRP mRNA molecules in the original extracted RNA sample and thus is semi-quantitative. It was observed that induction of angiogenesis by the fibroblast growth factor (FGF) induced the expression of h-SDRP - see Figure 6.

### Reverse transcription-polymerase chain reaction (Poly-A-PCR):

Poly-A-PCR was performed on total RNA extracted from human cell lines or tissues. 1 µg of total RNA was reverse transcribed using random hexamers to prime cDNA synthesis in a total volume of 20µl.

### cDNA reaction:

4µl of fresh 1^{st} strand buffer containing cDNA lysis buffer. 1 x prime mix (oligo(dT)₁₅ primer from Boehringer Mannheim, UK) and RNase inhibitor was added to 1µg of RNA. Components were mixed, incubated on ice for 10 minutes to 2 hours and heated to 65°C for 1 minute to denature the RNA and then cooled briefly on ice. 0.5µl (100 units) Reverse Transcriptase (Gibco BRL) was added, mixed and the tube briefly centrifuged. The reactions were incubated for exactly 15 minutes at 37°C and heat inactivated at 65°C for 10 minutes, then placed on ice.

### Terminal Transferase Reaction:

5µl of 2 x Tail and 0.5µl (10 units) of Terminal Transferase (Gibco BRL) were added and the samples were incubated for 15-60 minutes at 37°C. After the final denaturation at 65°C for 10 minutes, the samples were stored at -20°C or placed on ice to be used immediately for PCR reactions.

### Conditions for PCR reaction:

Obtained cDNA was used as a template for PCR reactions. PCR reaction mix was prepared as follows:

| Component | total volume added (µl) |
|---|---|
| 10 x TAQ Buffer (Boehringer Mannheim) | 10 |
| oligo (0.5-2.0 OD units/ml) Kvd or Not 1 | 1 |
| 25mM dNTPs | 1 |
| TAQ polymerase (Boehringer Mannheim) | 1 |
| cDNA template | 1-5 |
| ddH₂O | up to 100 |

Standard sequence of cycles for Poly-A-PCR is as follows:

| | |
|---|---|
| 25 cycles | 94°C for 1 minute, 42°C for 2 minutes, 72°C for 6 minutes. |

This was linked to:

| | |
|---|---|
| 25 cycles | 94°C for 1 minute, 42°C for 1 minute, 72°C for 2 minutes. |

### Ethidium bromide quantification of DNA and RNA solution:

Estimates of nucleic acid concentration were also obtained by visual comparison, through ethidium bromide staining of agarose gels containing aliquots of commercially quantified DNA and RNA (Gibco BRL).

### Southern blotting of DNA:

The gel was run according to standard methods and photographed. Next the gel was soaked in denaturation solution (0.5M NaOH, 1.5M NaCl) for 30 minutes. The gel was rinsed with distilled water, transferred to neutralising buffer (1.5M NaCl. 0.5M Tris HCl pH7.0) and soaked for 30 minutes at room temperature. Next the gel was soaked for 30 minutes at room temperature in 20X SSC buffer, with slow shaking. The gel then was carefully placed on a piece of Whatman 3MM paper, the same size as the gel and a pre-soaked nylon membrane (Hybond-N, Amersham) was put on top. On top of this were placed sheets of pre-wetted 3MM Whatman paper. The buffer tray was filled with transfer buffer (20x SSC) and transfer was started by connecting the gel stack with the buffer tray using the pre-cut "buffer wick", pre-soaked in transfer buffer. This was then left overnight or until all DNA from the gel was transferred onto the membrane. The membrane was then removed and gently washed in 2x SSC buffer for 10 minutes to remove agarose from the membrane. Next the membrane was exposed to a source of UV light for a total dose of 1200mJ/cm, to cross-link DNA to the filter.

### Prehybridisation of DNA filters:

Prehybridisation of the nylon membrane was carried out in 25 ml of hybridisation solution (5xSSPE, 5x Denhardt solution, 0.5% (w/v) SDS) at 65°C for 1-2 hours in rotating tubes in a hybridisation oven (TECHNE).

### Radiolabelling of DNA probes:

The radiolabelling of DNA probe S 112506 (SEQ ID No. 1) was performed using the Rediprime-Random Primer Labelling system from Amersham. To the premixed buffer and DNA polymerase solution, denatured DNA template (50ng) in a total volume of 12µl was added. The sample of DNA was denatured by heating to 90-95°C in a boiling water bath. 2µl of [³²P] (Amersham) was added to the probe/buffer mix and the samples were incubated at 37°C for 30 minutes. The reaction was stopped by adding 20µl of 1x STE buffer (100mM NaCl, 20mM Tris HCl pH7.5, 10mM EDTA). The probe was purified by passing the reaction mixture through a NucTrap probe push column (Stratagene) according to the manufacturer's instructions.

Briefly the column was first equilibrated with 70µl of STE buffer. The radiolabelled probe was made to a final volume of 60µl with STE buffer and then passed through the column. The probe was eluted from the column with 70µl of STE followed by a volume of air, and collected in an eppendorf tube.

The probe was denatured by heating in a boiling water bath for 10 minutes and chilled immediately on ice. The radioactive probe was mixed with 10ml of hybridisation solution and added to the tube with the filter. The filter was hybridised overnight at 65°C.

### Washing the filter:

Following hybridisation, the membrane was washed twice for 10 minutes at room temperature in 2x SSPE, 0.1% (w/v) SDS solution. The membrane was then washed with 1x SSPE, 0.1% (w/v) SDS for 15 minutes at 65°C. Washings were repeated until the background diminished. If required the membrane was washed in 0.1x SSPE, 0.1% (w/v) SDS for 10 minutes at 65°C. The membrane was then covered with clingfilm and was subjected to autoradiography.

### Autoradiography:

Filters were exposed to Kodak X-ray film at -70°C. The film was developed using Kodak developing system. Alternatively filters were developed after exposition on phospho-imager plate.

### Poly-A PCR analysis:

The expression of h-SDRP was investigated in bovine and human endothelial cells stimulated by different growth factors (figure 6).The figure shows the pattern of expression of the h-SDRP gene and a human osteonectin (a gene known to be expressed in endothelial cells) determined by the Poly-A PCR method using RNA extracted from bovine (baec) and human (heaec) endothelial cells. Cells in culture were treated, with appropriate non-treated controls, by different growth factors (basic fibroblast growth factor (bFGF), human growth factor (hGF) and VEGF) for different periods of time (24 and 48 hours) and RNA was extracted. Poly-A PCR products from treated samples were run with non-treated controls.

Figure 6A shows the ethidium bromide stained gel with 10µl of Poly-A PCR samples loaded. Figure 6B represents the equivalent Southern blot probed with 0.5 µg clone S 112506 (SEQ ID No. 1) and developed after 5 hour exposure on a phospho-imager. Figure 6C shows the ethidium bromide stained gel run with aliquots from the same Poly-A PCR samples as in (A). Figure 6D represents the equivalent Southern blot probed with human osteonectin and developed after I hour exposure.

Endothelial cells were chosen because Notch has been shown to be involved in angiogenesis. Comparison of non-treated and treated samples following probing with 3' specific h-SDRP probe (S112506) showed a change of gene expression in the bovine endothelial cells stimulated by bFGF for 24 hours. No change in expression was observed in cells treated for longer periods with bFGF before harvesting, or for cells treated with hGF or VEGF (B). As a control for the Poly-A PCR amplification method, hybridisation of the same Poly-A PCR samples with human osteonectin (a gene known to be expressed in endothelial cells) was performed (D). In most of the samples hybridisation to the human osteonectin was observed. Only one sample - the bovine endothelial control cells, grown for 48 hours (baec -hGF 48 h) failed with detection of the osteonectin gene.

The Poly-A PCR method was also applied to six human tumour cell line samples. The results indicated that only the rhabdomylosarcoma Rh18 represented a strong positive signal in response to the h-SDRP probe, despite comparable loadings. The rest of the tumour samples displayed only weak hybridisation signals. The lack of hybridisation to the negative control (sonicated λ DNA) indicated that the hybridisation stringency was such that the level of non-specific background was minimal. Both the undifferentiated and differentiated mouse C2C12 skeletal muscle samples showed a strong positive hybridisation signal in response to the human Su(dx) radioactive probe.

The pattern of the h-SDRP expression was also investigated in the human teratocarcinoma cell line samples. Cells were treated by retinoic acid to stimulate their differentiation. The Southern hybridisation result suggested that the h-SDRP gene is expressed in all the teratocarcinoma samples. A significant change of the levels of the h-SDRP expression on differentiation of these cells was not observed. The h-SDRP radioactive probe did not hybridise to the sonicated λ DNA control confirming the specificity of the method.

### SEQUENCES

SEQ ID NO. 1 – S112506
TTCATGGAAAGTTTATCGATACTGGTTTCTCTTTACCATTCTACAAGCGTA
TGTTAAGTAAAAACTTACTATTAAGGATTTGGAATCTATTGATACTGAAT
TTTATAACTCCCTTATCTGGATAAGAGATAACAACATTGAAGAATGTGGCT
TAGAAATGTACTTTTCTGTTGACATGGAGATTTTGGGAAAAGTTACTTCAC
ATGACCTGAAGTTGGGAGGTTCCAATATTCTGGTGACTGAGGAGAACAAA
GATGAATATATTGGTTTAATGACAGAATGGCGTTTTTCTCGAGGAGTACAA
GAACAGACCAAAGCTTTCCTTGATGGTTTAATGAAGTTGTTCCTCTTCAG
TGGCTACAGTACTTCGATGAAAAAGAATTAGAGGTTATGTTGTGTGGCAT
GCAGGAGGTTGACTTGGCAGATTGGCAGAGAAATACTGTTTATCGACATT
ATACAAGAAACAGCAAGCAAATCATTTGGTTTTGGCAGTTTGTGAAAGAG
ACAGACAATGAAGTAAGAATGCGACTATTGCAGTTCGTCACTGGAACCTG
CCGTTTACCTCTAGGAGGATTTGCTGAGCTCATGGGAAGTAATGGGCCTC
AAAAGTTTTGCATTGAAAAAGTTGGCAAAGACACTTGGTTACCAAGAAGC
CATACATGTTTTAATCGCTTGGATCTACCACCATATAAGAGTTATGAACAA
CTAAAGGAAAAACTTCTTTTTGCAATAGAAGAGACAGAGGGATTTGGACA
AGAATGAATGTGGCTTCTTATTTTGGAGGAGCTCTTGCATTTAAATACCCC
AGCCAAGAAAAATTGCACAGATAGTGTATATAAGCTGTTCATTCTGTACA
GTGAATTTTCCGAACCTCTCAAAGTATGTTTTCCGTTCTTCCACAGAAATA
TGCAAAACAGTTCATCCTTTTCTACTTTATTTATTGTTCCCTTGAAATGACT
GACCAGGAAAAAGATCATCCTTAAATTTTGAAGCAAGTGAGAGACTTTAT
TAAAAATACATATATATCTATATAAACATATATGATAGTGGCTCTAGTTTT
ATAGAGCTCCAAGTGTATTAAACATGACAGCCATTCATTCATAAAGATCT
GGATTTGCTTTACCTTGTTAATATTATCTAGGGGAAAAAGTGCAAATTGCT
CCATGTTCTTCTCTCCCTTATGTAACATCTCCTGAGGGTGTTTAGTTGCATG
GCTGTTCAGAAAGGTATTAAGGGCTTAGGCCAAATCTTACTTTGAGTATGT
TAAAAAAAAAAAAATGCTGCTGGCTTTTCTGAAGACAGGTGCTTGAACTT

GTCAGTTTGTTTTAAATAAATACAATAGTTGAAAATTTAAAAAAAAAAAA
AAAAAAAAAAAAAAAAAAAAAAA

SEQ ID No. 2 – 8IIIa
GATAATCATGTACCTACAAGCACTCTAGTCCAAAACTCATGCTGCTCGTAT
GTAGTTAATGGAGACAACACACCTTCATCTCCGTCTCAGGTTGCTGCCAGA
CCCAAAAATACACCAGCTCCAAAACCACTCGCATCTGAGCCTGCCGATGA
CACTGTTAATGGAGAATCATCCTCATTTGCACCAACTGATAATGCGTCTGT
CACGGGTACTCCAGTAGTGTCTGAAGAAAATGCCTTGTCTCCAAATTGCA
CTAGTACTACTGTTGAAGATCCTCCAGTTCAAGAAATACTGACTTCCTCAG
AAAACAATGAATGTATTCCTTCTACCAGTGCAGAATTGGAATCTGAAGCT
AGAAGTATATTAGAGCCTGACACCTCTAATTCTAGAAGTAGTTCTGCTTTT
GAAGCAGCCAAATCAAGACAGCCAGATGGGTGTATGGATCCTGTACGGCA
GCAGTCTGGGAATGCCAACACAGAAACCTTGCCATCAGGGTGGAACAA
AGAAAAGATCCTCATGGTAGAACCTATTATGTGGATCATAATACTCGAAC
TACCACATGGGAGAGACCACAACCTTTACCTCCAGGTTGGGAAAGAAGAG
TTGATGATCGTAGAAGAGTTTATTATGTGGATCATAACACCAGAACAACA
ACGTGGCAGCGGCCTACCATGGAATCTGTCCGAAATTTTGAACAGTGGCA
ATCTCAGCGGAACCAATTGCAGGGAGCTATGCAACAGTTTAACCAACGAT
ACCTCTATTCGGCTTCAATGTTAGCTGCAGAAAATGACCCTTATGGACCTT
TGCCACCAGGCTGGGAAAAAAGAGTGGATTCAACAGACAGGGTTTACTTT
GTGAATCATAACACAAAAACAACCCAGTGGGAAGATCCAAGAACTCAAG
GCTTACAGAATGAAGAACCCCTGCCAGAAGGCTGGGAAATTAGATATACT
CGTGAAGGTGTAAGGTACTTTGTTGATCATAACACAAGAACAACAACATT
CAAAGATCCTCGCAATGGGAAGTCATCTGTAACTAAAGGTGGTCCACAAA
TTGCTTATGAACGCGGCTTTAGGTGGAAGCTTGCTCACTTCCGTTATTTGT
GCCAGTCTAATGCACTACCTAGTCATGTAAAGATCAATGTGTCCCGGCAG
ACATTGTTTGAAGATTCCTTCCAACAGATTATGGCATTAAAACCCTATGAC
TTGAGGAGGCGCTTATATGTAATATTTAGAGGAGAAGAAGGACTTGATTA
TGGTGGCCTAGCGAGAGAATGGTTTTTCTTGCTTTCACATGAAGTTTTGAA

CCCAATGTATTGCTTATTTGAGTATGCGGGCAAGAACAACTATTGTCTGCA
GATAAATCCAGCATCAACCATTAATCCAGACCATCTTTCATACTTCTGTTT
CATTGGTCGTTTTATTGCCATGGCACTATTTCATGGAAAGTTTATCGATAC
TGGTTTCTCTTTACCATTCTACAAGCGTATGTTAAGTAAAAAACTTACTAT
TAAGGATTTGGAATCTATTGATACTGAATTTTATAACTCCCTTATCTGGAT
AAGAGATAACAACATTGAAGAATGTGGCTTAGAAATGTACTTTTCTGTTG
ACATGGAGATTTTGGGAAAAGTTACTTCACATGACCTGAAGTTGGGAGGT
TCCAATATTCTGGTGACTGAGGAGAACAAAGATGAATATATTGGTTTAAT
GACAGAATGGCGTTTTTCTCGAGGAGTACAAGAACAGACCAAAGCTTTCC
TTGATGGTTTTAATGAAGTTGTTCCTCTTCAGTGGCTACAGTACTTCGATG
AAAAAGAATTAGAGGTTATGTTGTGTGGCATGCAGGAGGTTGACTTGGCA
GATTGGCAGAGAAATACTGTTTATCGACATTATACAAGAAACAGCAAGCA
AATCATTTGGTTTTGGCAGTTTGTGAAAGAGACAGACAATGAAGTAAGAA
TGCGACTATTGCAGTTCGTCACTGGAACCTGCCGTTTACCTCTAGGAGGAT
TTGCTGAGCTCATGGGAAGTAATGGGCCTCAAAAGTTTTGCATTGAAAAA
GTTGGCAAAGACACTTGGTTACCAAGAAGCCATACATGTTTTAATCGCTTG
GATCTACCACCATATAAGAGTTATGAACAACTAAAGGAAAAACTTCTTTTT
GCAATAGAAGAGACAGAGGGATTTGGACAAGAATGAATGTGGCTTCTTAT
TTTGGAGGAGCTCTTGCATTTAAATACCCCAGCCAAGAAAAATTGCACAG
ATAGTGTATATAAGCTGTTCATTCTGTACAGTGAATTTTCCGAACCTCTCA
AAGTATGTTTTCCGTTCTTCCACAGAAATATGCAAAACAGTTCATCCTTTT
CTACTTTATTTATTGTTCCCTTGAAATGACTGACCAGGAAAAAGATCATCC
TTAAATTTTGAAGCAAGTGAGAGACTTTATTAAAAATACATATATATCTAT
ATAAACATATATGATAGTGGCTCTAGTTTTATAGAGCTCCAAGTGTATTAA
ACATGACAGCCATTCATTCATAAAGATCTGGATTTGCTTTACCTTGTTAAT
ATTATCTAGGGGAAAAAGTGCAAATTGCTCCATGTTCTTCTCTCCCTTATG
TAACATCTCCTGAGGGTGTTTAGTTGCATGGCTGTTCAGAAAGGTATTAAG
GGCTTAGGCCAAATCTTACTTTGAGTATGTTAAAAAAAAAAAAATGCTGC
TGGCTTTTCTGAAGACAGGTGCTTGAACTTGTCAGTTTGTTTTAAATAAAT

ACAATAGTTGAAAATTTAAAAAAAAAAAAAAAAAAAAAAAAAAAAAAA
AAA

SEQ ID NO. 3 – 8IIIa
DNHVPTSTLVQNSCCSYVVNGDNTPSSPSQVAARPKNTPAPKPLASEPADDT
VNGESSSFAPTDNASVTGTPVVSEENALSPNCTSTTVEDPPVQEILTSSENNECI
PSTSAELESEARSILEPDTSNSRSSSAFEAAKSRQPDGCMDPVRQQSGNANTET
LPSGWEQRKDPHGRTYYVDHNTRTTTWERPQPLPPGWERRVDDRRRVYYV
DHNTRTTTWQRPTMESVRNFEQWQSQRNQLQGAMQQFNQRYLYSASMLAA
ENDPYGPLPPGWEKRVDSTDRVYFVNHNTKTTQWEDPRTQGLQNEEPLPEG
WEIRYTREGVRYFVDHNTRTTTFKDPRNGKSSVTKGGPQIAYERGFRWKLAH
FRYLCQSNALPSHVKINVSRQTLFEDSFQQIMALKPYDLRRRLYVIFRGEEGL
DYGGLAREWFFLLSHEVLNPMYCLFEYAGKNNYCLQINPASTINPDHLSYFCF
IGRFIAMALFHGKFIDTGFSLPFYKRMLSKKLTIKDLESIDTEFYNSLIWIRDNN
IEECGLEMYFSVDMEILGKVTSHDLKLGGSNILVTEENKDEYIGLMTEWRFSR
GVQEQTKAFLDGFNEVVPLQWLQYFDEKELEVMLCGMQEVDLADWQRNTV
YRHYTRNSKQIIWFWQFVKETDNEVRMRLLQFVTGTCRLPLGGFAELMGSN
GPQKFCIEKVGKDTWLPRSHTCFNRLDLPPYKSYEQLKEKLLFAIEETEGFGQ
E

SEQ ID No. 4 – h-SDRP
GGCACGAGCGGCACGAGTGAAAGAGGGAATCGTGTCTTACTCATCTTTGT
ATCCCCAGTGTCTAGCAGTTCCTGATACATAGTTTTAGCTGAATTTTGGGA
CATGGCCACTGCTTCACCAAGGTCTGATACTAGTAATAACCACAGTGGAA
GGTTGCAGTTACAGGTAACTGTTTCTAGTGCCAAACTTAAAAGAAAAAAG
AACTGGTTCGGAACAGCAATATATACAGAAGTAGTTGTAGATGGAGAAAT
TACGAAAACAGCAAAATCCAGTAGTTCTTCTAATCCAAAATGGGATGAAC
AGCTAACTGTAAATGTTACGCCACAGACTACATTGGAATTTCAAGTTTGGA
GCCATCGCACTTTAAAAGCAGATGCTTTATTAGGAAAAGCAACGATAGAT
TTGAAACAAGCTCTGTTGATACACAATAGAAAATTGGAAAGAGTGAAAGA

ACAATTAAAACTTTCCTTGGAAAACAAGAATGGCATAGCACAAACTGGTG
AATTGACAGTTGTGCTTGATGGATTGGTGATTGAGCAAGAAAATATAACA
AACTGCAGCTCATCTCCAACCATAGAAATACAGGAAAATGGTGATGCCTT
ACATGAAAATGGAGAGCCTTCAGCAAGGACAACTGCCAGGTTGGCTGTTG
AAGGCACGAATGGAATAGATAATCATGTACCTACAAGCACTCTAGTCCAA
AACTCATGCTGCTCGTATGTAGTTAATGGAGACAACACACCTTCATCTCCG
TCTCAGGTTGCTGCCAGACCCAAAAATACACCAGCTCCAAAACCACTCGC
ATCTGAGCCTGCCGATGACACTGTTAATGGAGAATCATCCTCATTTGCACC
AACTGATAATGCGTCTGTCACGGGTACTCCAGTAGTGTCTGAAGAAAATG
CCTTGTCTCCAAATTGCACTAGTACTACTGTTGAAGATCCTCCAGTTCAAG
AAATACTGACTTCCTCAGAAAACAATGAATGTATTCCTTCTACCAGTGCAG
AATTGGAATCTGAAGCTAGAAGTATATTAGAGCCTGACACCTCTAATTCTA
GAAGTAGTTCTGCTTTTGAAGCAGCCAAATCAAGACAGCCAGATGGGTGT
ATGGATCCTGTACGGCAGCAGTCTGGGAATGCCAACACAGAAACCTTGCC
ATCAGGGTGGGAACAAAGAAAAGATCCTCATGGTAGAACCTATTATGTGG
ATCATAATACTCGAACTACCACATGGGAGAGACCACAACCTTTACCTCCA
GGTTGGGAAAGAAGAGTTGATGATCGTAGAAGAGTTTATTATGTGGATCA
TAACACCAGAACAACAACGTGGCAGCGGCCTACCATGGAATCTGTCCGAA
ATTTTGAACAGTGGCAATCTCAGCGGAACCAATTGCAGGGAGCTATGCAA
CAGTTTAACCAACGATACCTCTATTCGGCTTCAATGTTAGCTGCAGAAAAT
GACCCTTATGGACCTTTGCCACCAGGCTGGGAAAAAAGAGTGGATTCAAC
AGACAGGGTTTACTTTGTGAATCATAACACAAAAACAACCCAGTGGGAAG
ATCCAAGAACTCAAGGCTTACAGAATGAAGAACCCCTGCCAGAAGGCTGG
GAAATTAGATATACTCGTGAAGGTGTAAGGTACTTTGTTGATCATAACAA
GAACAACAACATTCAAAGATCCTCGCAATGGGAAGTCATCTGTAACTAAA
GGTGGTCCACAAATTGCTTATGAACGCGGCTTTAGGTGGAAGCTTGCTCA
CTTCCGTTATTTGTGCCAGTCTAATGCACTACCTAGTCATGTAAAGATCAA
TGTGTCCCGGCAGACATTGTTTGAAGATTCCTTCCAACAGATTATGGCATT
AAAACCCTATGACTTGAGGAGGCGCTTATATGTAATATTTAGAGGAGAAG
AAGGACTTGATTATGGTGGCCTAGCGAGAGAATGGTTTTTCTTGCTTTCAC

ATGAAGTTTTGAACCCAATGTATTGCTTATTTGAGTATGCGGGCAAGAACA
ACTATTGTCTGCAGATAAATCCAGCATCAACCATTAATCCAGACCATCTTT
CATACTTCTGTTTCATTGGTCGTTTATTGCCATGGCACTATTTCATGGAAA
GTTTATCGATACTGGTTTCTCTTTACCATTCTACAAGCGTATGTTAAGTAA
AAAACTTACTATTAAGGATTTGGAATCTATTGATACTGAATTTTATAACTC
CCTTATCTGGATAAGAGATAACAACATTGAAGAATGTGGCTTAGAAATGT
ACTTTTCTGTTGACATGGAGATTTTGGGAAAAGTTACTTCACATGACCTGA
AGTTGGGAGGTTCCAATATTCTGGTGACTGAGGAGAACAAAGATGAATAT
ATTGGTTTAATGACAGAATGGCGTTTTTCTCGAGGAGTACAAGAACAGAC
CAAAGCTTTCCTTGATGGTTTTAATGAAGTTGTTCCTCTTCAGTGGCTACA
GTACTTCGATGAAAAAGAATTAGAGGTTATGTTGTGTGGCATGCAGGAGG
TTGACTTGGCAGATTGGCAGAGAAATACTGTTTATCGACATTATACAAGA
AACAGCAAGCAAATCATTTGGTTTTGGCAGTTTGTGAAAGAGACAGACAA
TGAAGTAAGAATGCGACTATTGCAGTTCGTCACTGGAACCTGCCGTTTACC
TCTAGGAGGATTTGCTGAGCTCATGGGAAGTAATGGGCCTCAAAAGTTTT
GCATTGAAAAAGTTGGCAAAGACACTTGGTTACCAAGAAGCCATACATGT
TTTAATCGCTTGGATCTACCACCATATAAGAGTTATGAACAACTAAAGGA
AAAACTTCTTTTTGCAATAGAAGAGACAGAGGGATTTGGACAAGAATGAA
TGTGGCTTCTTATTTTGGAGGAGCTCTTGCATTTAAATACCCCAGCCAAGA
AAAATTGCACAGATAGTGTATATAAGCTGTTCATTCTGTACAGTGAATTTT
CCGAACCTCTCAAAGTATGTTTTCCGTTCTTCCACAGAAATATGCAAAACA
GTTCATCCTTTTCTACTTTATTTATTGTTCCCTTGAAATGACTGACCAGGAA
AAAGATCATCCTTAAATTTTGAAGCAAGTGAGAGACTTTATTAAAAATAC
ATATATATCTATATAAACATATATGATAGTGGCTCTAGTTTATAGAGCTC
CAAGTGTATTAAACATGACAGCCATTCATTCATAAAGATCTGGATTTGCTT
TACCTTGTTAATATTATCTAGGGGAAAAAGTGCAAATTGCTCCATGTTCTT
CTCTCCCTTATGTAACATCTCCTGAGGGTGTTTAGTTGCATGGCTGTTCAG
AAAGGTATTAAGGGCTTAGGCCAAATCTTACTTTGAGTATGTTAAAAAA
AAAAAATGCTGCTGGCTTTTCTGAAGACAGGTGCTTGAACTTGTCAGTTTG

TTTTAAATAAATACAATAGTTGAAAATTTAAAAAAAAAAAAAAAAAAAAAAAAAAA

AAAAAAAAAAAAAAAAA

SEQ ID No. 5 – h-SDRP
MATASPRSDTSNNHSGRLQLQVTVSSAKLKRKKNWFGTAIYTEVVVDGEITK
TAKSSSSSNPKWDEQLTVNVTPQTTLEFQVWSHRTLKADALLGKATIDLKQA
LLIHNRKLERVKEQLKLSLENKNGIAQTGELTVVLDGLVIEQENITNCSSSPTIE
IQENGDALHENGEPSARTTARLAVEGTNGIDNHVPTSTLVQNSCCSYVVNGD
NTPSSPSQVAARPKNTPAPKPLASEPADDTVNGESSSFAPTDNASVTGTPVVS
EENALSPNCTSTTVEDPPVQEILTSSENNECIPSTSAELESEARSILEPDTSNSRS
SSAFEAAKSRQPDGCMDPVRQQSGNANTETLPSGWEQRKDPHGRTYYVDHN
TRTTTWERPQPLPPGWERRVDDRRRVYYVDHNTRTTTWQRPTMESVRNFEQ
WQSQRNQLQGAMQQFNQRYLYSASMLAAENDPYGPLPPGWEKRVDSTDRV
YFVNHNTKTTQWEDPRTQGLQNEEPLPEGWEIRYTREGVRYFVDHNTRTTTF
KDPRNGKSSVTKGGPQIAYERGFRWKLAHFRYLCQSNALPSHVKINVSRQTL
FEDSFQQIMALKPYDLRRRLYVIFRGEEGLDYGGLAREWFFLLSHEVLNPMY
CLFEYAGKNNYCLQINPASTINPDHLSYFCFIGRFIAMALFHGKFIDTGFSLPFY
KRMLSKKLTIKDLESIDTEFYNSLIWIRDNNIEECGLEMYFSVDMEILGKVTSH
DLKLGGSNILVTEENKDEYIGLMTEWRFSRGVQEQTKAFLDGFNEVVPLQWL
QYFDEKELEVMLCGMQEVDLADWQRNTVYRHYTRNSKQIIWFWQFVKETD
NEVRMRLLQFVTGTCRLPLGGFAELMGSNGPQKFCIEKVGKDTWLPRSHTCF
NRLDLPPYKSYEQLKEKLLFAIEETEGFGQE

SEQ ID No. 6
GAA AGA GGG AAT CGT GTC TTA C

SEQ ID NO. 7
CGA TCA TCA ACT CTT CTT TCC C

## Claims

1. An *in vitro* method of screening for diseases caused by abnormal Notch signalling, the method comprising assaying for the expression of a nucleic acid encoding a human suppressor of deltex related protein, in a sample which has been obtained from an individual, wherein increased expression of the nucleic acid is associated with cell proliferation.

2. A method according to claim 1, wherein the nucleic acid has at least 60% homology to the sequence provided as SEQ ID NO. 4.

3. A method according to claim 1, wherein the nucleic acid has at least 70% homology to the sequence provided as SEQ ID NO. 4.

4. A method according to claim 1, wherein the nucleic acid has at least 80% homology to the sequence provided as SEQ ID NO. 4.

5. A method according to claim 1, wherein the nucleic acid has at least 90% homology to the sequence provided as SEQ ID NO. 4.

6. A method according to claim 1, wherein the nucleic acid comprises a nucleic acid sequence shown in SEQ ID NO. 4.

7. An *in vitro* method of screening for diseases caused by abnormal Notch signalling, the method comprising assaying for human suppressor of deltex related protein, in a sample which has been obtained from an individual, wherein an increased level of human suppressor of deltex related protein is associated with cell proliferation.

8. A method according to claim 7, wherein the protein has at least 60% homology to the sequence provided as SEQ ID NO. 5.

9. A method according to claim 7, wherein the protein has at least 70% homology to the sequence provided as SEQ ID NO. 5.

10. A method according to claim 7, wherein the protein has at least 80% homology to the sequence provided as SEQ ID NO. 5.

11. A method according to claim 7, wherein the protein has at least 90% homology to the sequence provided as SEQ ID NO. 5.

12. A method according to claim 7, wherein the protein comprises amino acid sequence shown in SEQ ID NO. 5.

13. A method according to any preceding claim, wherein the diseases which can be screened for comprise oncogenesis, colon cancer, cervical cancer, breast cancer, increased angiogenesis, squamous adenocarcinoma, seminoma, melanoma, lung cancer, dementia, CADASIL, wound healing, rheumatoid arthritis, and vascular diseases, such as arteriosclerosis.

14. A method according to claim 13, wherein the diseases which can be screened for include increased angiogenesis and breast cancer.

## Patentansprüche

1. Ein *in-vitro*-Verfahren zum Screening auf Krankheiten, welche durch einen anomalen Notch-Signalübertragungsweg verursacht werden, wobei das Verfahren das Untersuchen der Expression einer Nukleinsäure umfasst, die für einen humanen Suppressor des Deltex-verwandten Proteins codiert, in einer Probe, die von einem Individuum erhalten worden ist, wobei eine erhöhte Expression der Nukleinsäure in Zusammenhang steht mit Zellproliferation.

2. Ein Verfahren nach Anspruch 1, worin die Nukleinsäure eine Homologie von mindestens 60% zu der in SEQ ID NO. 4 bereitgestellten Sequenz aufweist.

3. Ein Verfahren nach Anspruch 1, worin die Nukleinsäure eine Homologie von mindestens 70% zu der in SEQ ID NO. 4 bereitgestellten Sequenz aufweist.

4. Ein Verfahren nach Anspruch 1, worin die Nukleinsäure eine Homologie von mindestens 80% zu der in SEQ ID NO. 4 bereitgestellten Sequenz aufweist.

5. Ein Verfahren nach Anspruch 1, worin die Nukleinsäure eine Homologie von mindestens 90% zu der in SEQ ID NO. 4 bereitgestellten Sequenz aufweist.

6. Ein Verfahren nach Anspruch 1, worin die Nukleinsäure eine Nukleinsäuresequenz, welche in SEQ ID NO. 4 gezeigt wird, umfasst.

7. Ein *in-vitro*-Verfahren zum Screening auf Krankheiten, welche durch einen anomalen Notch-Signalübertragungsweg verursacht werden, wobei das Verfahren das Untersuchen auf einen humanen Suppressor des Deltex-verwandten Proteins umfasst, in einer Probe, die von einem Individuum erhalten worden ist, wobei ein erhöhter Spiegel des humanen Suppressors des Deltex-verwandten Proteins in Zusammenhang steht mit Zellproliferation.

8. Ein Verfahren nach Anspruch 7, worin das Protein eine Homologie von mindestens 60% zu der als SEQ ID NO. 5 bereitgestellten Sequenz aufweist.

9. Ein Verfahren nach Anspruch 7, worin das Protein eine Homologie von mindestens 70% zu der als SEQ ID NO. 5 bereitgestellten Sequenz aufweist.

10. Ein Verfahren nach Anspruch 7, worin das Protein eine Homologie von mindestens 80% zu der als SEQ ID NO. 5 bereitgestellten Sequenz aufweist.

11. Ein Verfahren nach Anspruch 7, worin das Protein eine Homologie von mindestens 90% zu der als SEQ ID NO. 5 bereitgestellten Sequenz aufweist.

12. Ein Verfahren nach Anspruch 7, worin das Protein die Aminosäuresequenz, welche in SEQ ID NO. 5 gezeigt wird, umfasst.

13. Ein Verfahren nach einem der vorhergehenden Ansprüche, worin die Krankheiten, auf die hin ein Screening durchgeführt werden kann, Onkogenese, Kolonkrebs, Zervikalkrebs, Brustkrebs, erhöhte Angiogenese, Plattenepithel-Adenokarzinom, Seminom, Melanom, Lungenkrebs, Demenz, CADASIL, Wundheilung, rheumatoide Arthritis und vaskuläre Erkrankungen, so wie Arteriosklerose, umfassen.

14. Ein Verfahren nach Anspruch 13, worin die Krankheiten, auf die hin ein Screening durchgeführt werden kann, erhöhte Angiogenese und Brustkrebs einschließen.

## Revendications

1. Méthode de dépistage *in vitro* de maladies causées par une signalisation Notch anormale, la méthode comprenant doser l'expression d'un acide nucléique codant un suppresseur humain de protéine apparentée à deltex dans un échantillon qui a été obtenu auprès d'un individu, où une expression accrue de l'acide nucléique est associée à une prolifération cellulaire.

2. Méthode selon la revendication 1, dans laquelle l'acide nucléique a au moins 60% d'homologie avec la séquence donnée en tant que SEQ ID NO.4.

3. Méthode selon la revendication 1, dans laquelle l'acide nucléique a au moins 70% d'homologie avec la séquence donnée en tant que SEQ ID NO.4.

4. Méthode selon la revendication 1, dans laquelle l'acide nucléique a au moins 80% d'homologie avec la séquence donnée en tant que SEQ ID NO.4.

5. Méthode selon la revendication 1, dans laquelle l'acide nucléique a au moins 90% d'homologie avec la séquence donnée en tant que SEQ ID NO.4.

6. Méthode selon la revendication 1, dans laquelle l'acide nucléique comprend une séquence d'acide nucléique présentée dans la SEQ ID NO.4.

7. Méthode de dépistage *in vitro* de maladies causées par une signalisation Notch anormale, la méthode comprenant doser un suppresseur humain de protéine apparentée à deltex dans un échantillon qui a été obtenu auprès d'un individu, où un niveau accru de suppresseur humain de protéine apparentée à deltex, est associé à une prolifération cellulaire.

8. Méthode selon la revendication 7, dans laquelle la protéine a au moins 60% d'homologie avec la séquence donnée en tant que SEQ ID NO.5.

9. Méthode selon la revendication 7, dans laquelle la protéine a au moins 70% d'homologie avec la séquence donnée en tant que SEQ ID NO.5.

10. Méthode selon la revendication 7, dans laquelle la protéine a au moins 80% d'homologie avec la séquence donnée en tant que SEQ ID NO.5.

11. Méthode selon la revendication 7, dans laquelle la protéine a au moins 90% d'homologie avec la séquence donnée en tant que SEQ ID NO.5.

12. Méthode selon la revendication 7, dans laquelle la protéine comprend une séquence d'acides aminés présentée dans la SEQ ID NO.5.

13. Méthode selon l'une quelconque des revendications précédentes, dans laquelle les maladies qui peuvent être dépistées comprennent l'oncogenèse, le cancer du côlon, le cancer du col de l'utérus, le cancer du sein, l'angiogenèse augmentée, l'adénocarcinome squameux, le séminome, le mélanome, le cancer du poumon, la démence, CADASIL, la cicatrisation des plaies, l'arthrite rhumatoïde et les maladies vasculaires telles que l'artériosclérose.

14. Méthode selon la revendication 13, dans laquelle les maladies qui peuvent être dépistées comprennent l'angiogenèse augmentée et le cancer du sein.
